# EUROPEAN PATENT APPLICATION

(11) **EP 2 415 686 A1**
(43) Date of publication of application: **08.02.2012**
(21) Application number: 10758871.7
(22) Date of filing: 01.04.2010
(51) Int. Cl.: B65D 41/34, A61J 1/05, B65D 41/04

(54) **CONTAINER FOR TWO-COMPONENT MEDICAMENT**

(30) Priority: 02.04.2009 JP 2009090182
(71) Applicant: Senju Pharmaceutical Co., Ltd., Osaka-shi, Osaka 541-0046 (JP); Hanshin Kasei Co., Ltd., Toyama-shi, Toyama 939-8183 (JP)
(72) Inventor: OTSUKA Tadashi, Kobe-shi Hyogo 651-2241 (JP); FUJIMOTO Takashi, Toyama-shi Toyama 939-8183 (JP)
(74) Representative: Knoop, Philipp
(86) International application number: PCT/JP2010/056028
(87) International publication number: WO 2010/114100

(57) **Abstract**

The present invention provides a two-drug pharmaceutical container in which it is possible to remove the cap without loosening its nozzle cap and container main body when its cap is turned in the opening direction at the time of administering eye drops or the like.

The container in which, on an outer surface of a nozzle section 12 of a container main body 1, a small diameter annular section 14 on which upper stage external ratchet teeth 16 are formed and a large diameter annular section 15 on which lower stage external ratchet teeth 17 are formed are provided, and in a nozzle cap 2 whose body section 22 and sealing ring part 23 are jointed together so as to be breakable, upper stage internal ratchet teeth 28 and lower stage internal ratchet teeth 29 are provided on the inner surfaces of the body section 22 and the sealing ring part 23, and the container is configured so that only the lower stage internal and external ratchet teeth 17 and 29 engage with each other to prevent turning in the opening direction in a closed state before use, and moreover, the upper stage internal and external ratchet teeth 16 and 28 engage with each other to prevent turning in the opening direction in a state in which the container main body is closed with the nozzle cap 2 without the sealing ring part 23.

## Description

### [Technical Field]

Thepresentinvention relatesto a two-drug pharmaceutical container whose container main body containing a first ingredient is opened to add a second ingredient thereinto, and the container main body is thereafter again closed to be used for the purpose of eye drops or the like. In particular, the present invention relates to a two-drug pharmaceutical container in which a ratchet mechanism is provided at its nozzle cap and container main body in order to prevent inadvertent opening by a user, to prevent the nozzle cap from being loosened in the opening direction.

### [Background Art]

Conventionally, as a two-drug pharmaceutical container whose container main body containing a first ingredient is opened to add a second ingredient thereinto, and the container main body is thereafter again closed to be used for the purpose of eye drops or the like, for example, a two-drug pharmaceutical container in which a nozzle cap is screwed onto a container main body, a medicinal solution extracting orifice is provided at a head section of the nozzle cap, and a cap is screwed onto the head section, is known.

### [Prior Art Document]

### [Patent Document]

[Patent Document 1] Japanese Published Unexamined Patent Application-Hei-7-257610

### [Summary of the Invention]

### [Problems to be Solved by the Invention]

However, since the direction of screwing the container main body and the nozzle cap, and the nozzle cap and the cap are the same in the conventional two-drug pharmaceutical container, the nozzle cap is rotated in the opening direction to remove the nozzle cap from the container main body to add a second ingredient into the case main body, and the nozzle cap is rotated in the closing direction to close the container main body, and thereafter, when the cap is rotated in the opening direction to remove the cap from the nozzle cap to administer eye drops or the like, the nozzle cap as well may be rotated in the opening direction at the same time in some cases, and the medicinal solution may spill out from the mouth portion of the loose container main body in some cases.

Conventionally, a container including a ratchet mechanism in order not to allow its container main body and cap to be opened is known. For example, there is disclosed a container which is configured so that ratchet teeth are respectively provided on the outer side of the terminal end of its cervical part of the container and on the inner side of the cap (Patent Document 1).

Then, a ratchet mechanism is expected to be used in order to prevent the nozzle cap from being loosened at the time of administering eye drops or the like. However, the container disclosed in Patent Document 1 is applicable as is to a single-drug pharmaceutical container, but is not applicable as is to a two-drug pharmaceutical container associated with an operation of removing the nozzle cap in order to add a second ingredient into the container main body containing a first ingredient.

The present invention has been achieved in consideration of the above-described circumstances. It is a primary object of the present invention to provide a two-drug pharmaceutical container in which a nozzle cap is screwed onto a container main body, a medicinal solution extracting orifice is provided at a head section of the nozzle cap, and a cap is screwed onto the head section, in the two-drug pharmaceutical container, the container main body and the nozzle cap are not loosened in any case until mixture of two medicinal solutions, these can be easily removed by breaking a ratchet section at the time of mixture, and further, when these are again closed after mixture, this portion is not easily opened thereafter, therefore, even when the cap is turned in the opening direction, the nozzle cap and the container main body are not loosened in any case, that keeps the closed state, which makes it possible to remove only the cap.

### [Means for Solving the Problem]

That is, the scope of the present invention is as follows.
[1] A two-drug pharmaceutical container including a container main body which is filled with a first ingredient, and provided with a male screw on an outer circumferential surface of a nozzle section,
   a nozzle cap which has a body section provided with a female screw screwed together with the male screw on an inner circumferential surface of its tube body, a head section including a medicinal solution extracting orifice communicated with an inside of the body section, and a sealing ring part provided via a plurality of breakable joining sections which are provided along a circumferential direction on a lower edge of the body section, and
   a cap screwed on so as to cover the head section of the nozzle cap, and in the container,
   a small diameter annular section with an outer diameter larger than an outer diameter of the male screw of the nozzle section and a large diameter annular section, which is on the lower side of the small diameter annular section, with an outer diameter larger than the outer diameter of the small diameter annular section are provided at a lower portion of the nozzle section of the container main body, and upper stage external ratchet teeth and lower stage external ratchet teeth are respectively provided on an outer circumferential surface of the small diameter annular section and an outer circumferential surface of the large diameter annular section, and on the other hand, upper stage internal ratchet teeth and lower stage internal ratchet teeth are respectively provided on an inner circumferential surface of a lower portion of the body section and an inner circumferential surface of the sealing ring part in the components of the nozzle cap,
   and in a closed state in which the female screw of the body section of the nozzle cap is screwed together with the male screw of the container main body to be turned up to a position at which the container main body is closed, although the upper stage internal ratchet teeth and the upper stage external ratchet teeth do not engage with each other, the lower stage internal ratchet teeth and the lower stage external ratchet teeth engage with each other to prevent turning in an opening direction, and moreover,
   in a closed state in which the female screw of the body section of the nozzle cap from which the sealing ring part is removed by breaking the joining sections of the nozzle cap, is screwed together with the male screw of the container main body, and the container main body is turned up to the position to be closed, the upper stage internal ratchet teeth and the upper stage external ratchet teeth engage with each other to prevent turning in the opening direction.
[2] In the two-drug pharmaceutical container according to [1], a downward protruding piece is provided on the lower edge of the body section and an upward protruding piece is provided so as to come close to the closing side in the circumferential direction from the downward protruding piece on an upper edge of the sealing ring part in the components of the nozzle cap, and the front part lower end of the downward protruding piece is extended downward from the rear part upper end of the upward protruding piece.
[3] The two-drug pharmaceutical container according to [1] or [2], the container is used for any purpose of eye drops, ear drops, and nose drops.
[4] A method for using the two-drug pharmaceutical container including the successive processes of
   in use of the two-drug pharmaceutical container according to any one of [1] to [3],
   operating the nozzle cap to turn in the opening direction to break the joining sections and removing the nozzle cap without the sealing ring part and the sealing ring part from the container main body,
   adding a second ingredient into the container main body and operating the nozzle cap to turn in the closing direction to close the container main body, and
   operating the cap to turn in the opening direction to remove the cap from the nozzle cap, and discharging a medicinal solution containing the first ingredient and the second ingredient from the medicinal solution extracting orifice.

### [Effects of the Invention]

According to the two-drug pharmaceutical container of the present invention, that is configured so that the lower stage internal ratchet teeth and the lower stage external ratchet teeth engage with each other to prevent turning in the opening direction before use, the container main body and the nozzle cap are not easily loosened in any case. Then, when the nozzle cap is operated to turn in the opening direction at the time of adding a second ingredient, the joining sections are broken while the lower stage internal ratchet teeth and the lower stage external ratchet teeth engage with each other to prevent turning in the opening direction, which makes it possible to remove the nozzle cap. Then, since the two-drug pharmaceutical container is configured so that a sealing ring part is removed from the container main body to add a second ingredient into the container main body containing a first ingredient, and thereafter, when the nozzle cap without a sealing ring part because of the breakage of the joining sections is operated to turn in the closing direction to close the container main body, and next the cap is operated to turn in the opening direction, the upper stage internal ratchet teeth and the upper stage external ratchet teeth engage with each other to prevent turning in the opening direction, the closed state between the nozzle cap and the container main body is kept, which makes it possible to remove only the cap.

### [Brief Description of the Drawings]

Fig. 1 is an exploded perspective view of a two-drug pharmaceutical container according to the present invention whose cap is not shown and whose nozzle cap is partially cut out.
Fig. 2A is a view of the container main body in Fig. 1 viewed from above. Fig. 2B is a view of the container main body in Fig. 1 viewed from the front.
Fig. 3A is a front half sectional view of the nozzle cap in Fig. 1. Fig. 3B is a view of the nozzle cap of Fig. 3A viewed from below.
Fig. 4 is a side view of the nozzle cap in Fig. 1.
Fig. 5A is a longitudinal sectional view showing the state of the container of Fig. 1 before use. Fig. 5B is a longitudinal sectional view of Fig. 5A taken along line A-A.
Fig. 6A is a longitudinal sectional view showing the state in which the container main body is closed with the nozzle cap from which a sealing ring part is removed by breaking joining sections. Fig. 6B is a cross sectional view of Fig. 6A taken along line B-B.

### [Embodiment for Carrying Out the Invention]

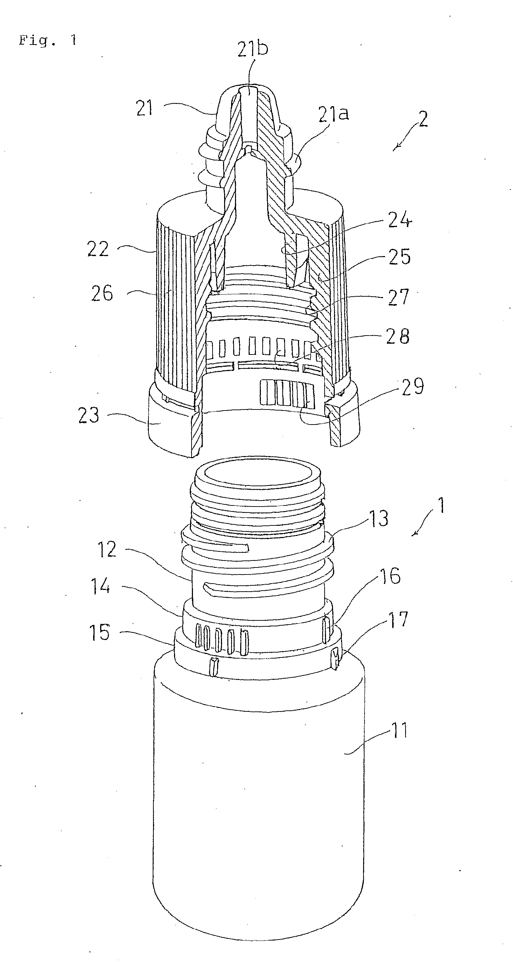

As shown in Figs. 1 to 6, a two-drug pharmaceutical container according to the present invention is composed of a container main body 1, a nozzle cap 2, and a cap 3 which are all made of synthetic resin, and can be suitably used for the purpose of, for example, eye drops, ear drops, nose drops, or the like.

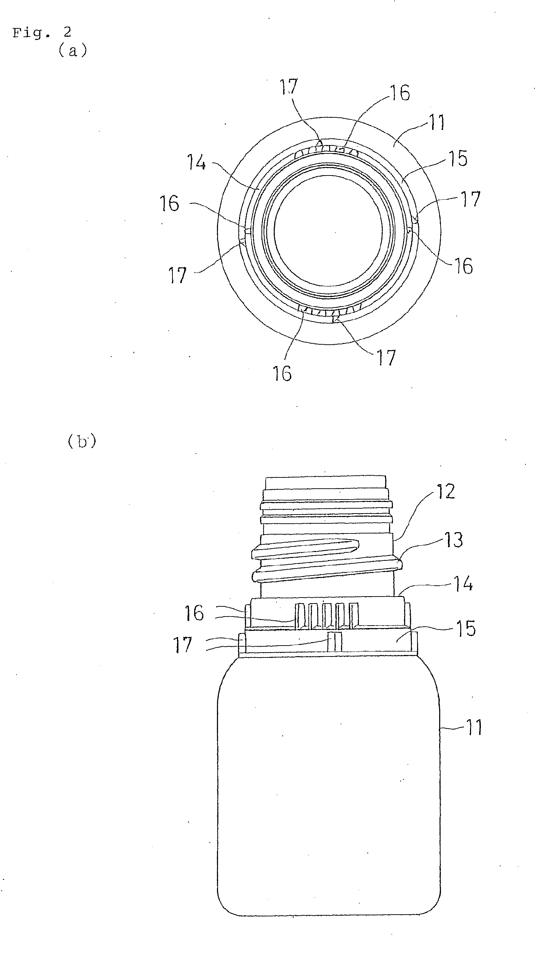

As shown in Figs. 1 and 2, in the container main body 1, a cylindrical body section 11 which is filled with a first ingredient (not shown), and the container main body 1 has a nozzle section 12 which is consecutively installed upward from the body section 11. A male screw 13 is provided on the outer circumferential surface of the nozzle section 12, and a small diameter annular section 14 with an outer diameter larger than an outer diameter of the male screw 13 and a large diameter annular section 15, which is on the lower side of the small diameter annular section 14, with an outer diameter larger than the outer diameter of the small diameter annular section 14 are consecutively installed to the lower portion of the nozzle section 12. Upper stage external ratchet teeth 16 and lower stage external ratchet teeth 17 are respectively provided on an outer circumferential surface of the small diameter annular section 14 and an outer circumferential surface of the large diameter annular section 15.

As shown in Fig. 2, the respective teeth composing the upper stage external ratchet teeth 16 are formed into the same shape that is saw-like, two groups of the teeth 16 in which five teeth are consecutively installed at regular pitches are provided so as to face each other on the outer circumferential surface of the small diameter annular section 14, and the two teeth 16 are provided so as to face each other at positions respectively shifted by approximately 90 degrees from these groups of the teeth 16 on the outer circumferential surface. Further, the respective teeth composing the lower stage external ratchet teeth 17 are formed into the same shape that is saw-like, and the four teeth 17 are provided at regular pitches on an outer circumferential surface of the large diameter annular section 15.

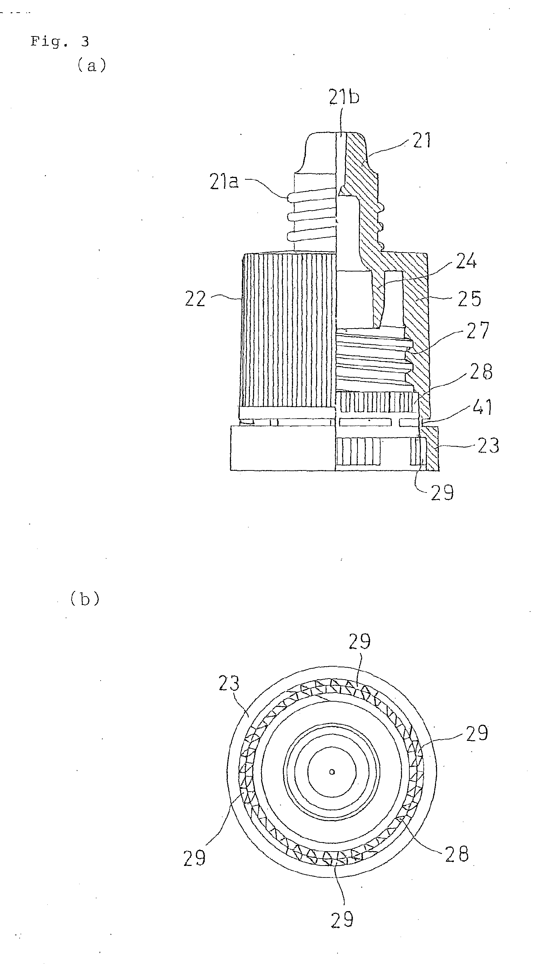

As shown in Figs. 1 and 3, the nozzle cap 2 forms a cylindrical shape where a head section 21, a body section 22, a sealing ring part 23 are integrally molded in its central axis direction. The body section 22 has a shorter internal tube 24 and a longer external tube 25 on the outer side of the internal tube 24, and an antislip knurl 26 and a female screw 27 screwed together with the male screw 13 of the nozzle section 12 are respectively provided on the outer circumferential surface of the external tube 25 and the inner circumferential surface of the external tube 25, and upper stage internal ratchet teeth 28 are provided at the lower portion of the inner circumferential surface of the external tube 25.

As shown in Fig. 3, the respective teeth composing the upper stage internal ratchet teeth 28 are formed into the same shape that is saw-like, and are provided in large numbers so as to go around the inner circumferential surface at regular pitches on the inner circumferential surface of the external tube 25.

The sealing ring part 23 has an outer diameter larger than the outer diameter of the external tube 25, and lower stage internal ratchet teeth 29 are provided on the inner circumferential surface of the sealing ring part 23. The sealing ring part 23 is jointed together with breakable joining sections 41 which is provided on the lower edge of the body section 22 along the circumferential direction through its lower edge, to be jointed together with the body section 22 (refer to Fig. 3).

As shown in Fig. 3, the respective teeth composing the lower stage internal ratchet teeth 29 are formed into the same shape that is saw-like, and four groups of the teeth 29 in which six teeth are consecutively installed at regular pitches are provided at regular pitches on the inner circumferential surface of the sealing ring part 23.

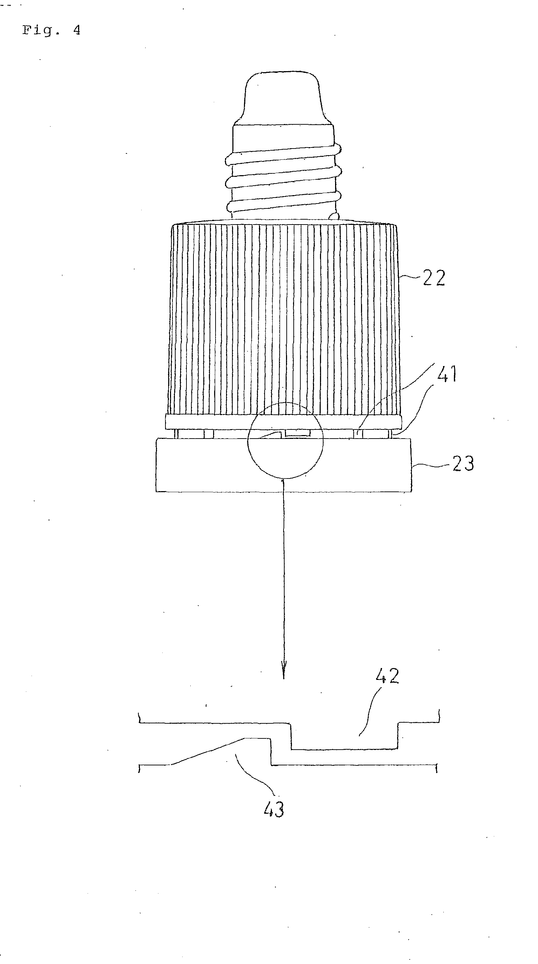

As shown in an enlarged view of Fig. 4, a downwardprotruding piece 42 is provided on the lower edge of the body section 22, and an upward protruding piece 43 is provided so as to come close to the closing side in the circumferential direction from the downward protruding piece 42 (on the left side from the downward protruding piece 42 in Fig. 4) on the same circumference on an upper edge of the sealing ring part 23 in the components of the nozzle cap 2, and the front part lower end of the downward protruding piece 42 is extended downward from the rear part upper end of the upward protruding piece 43.

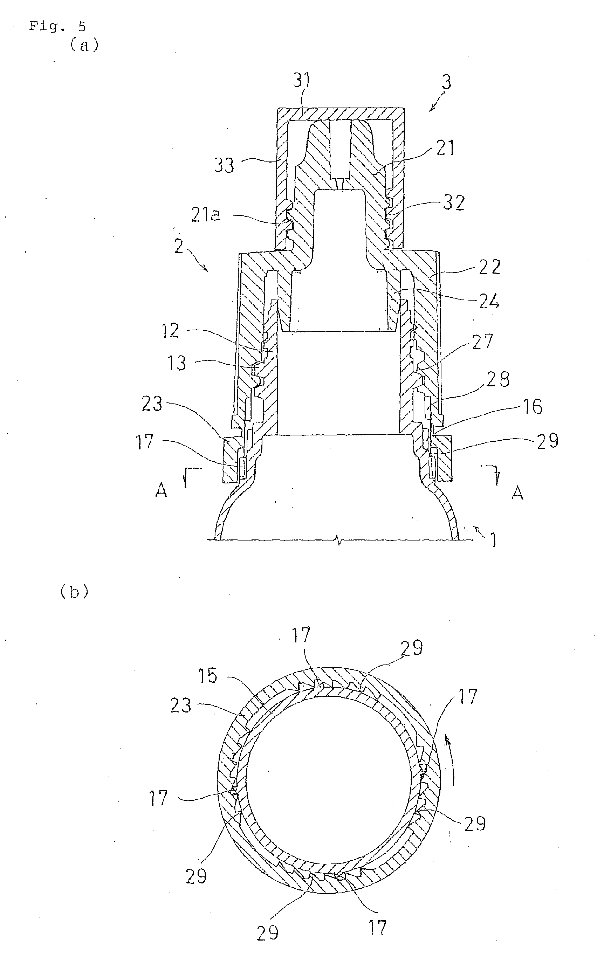

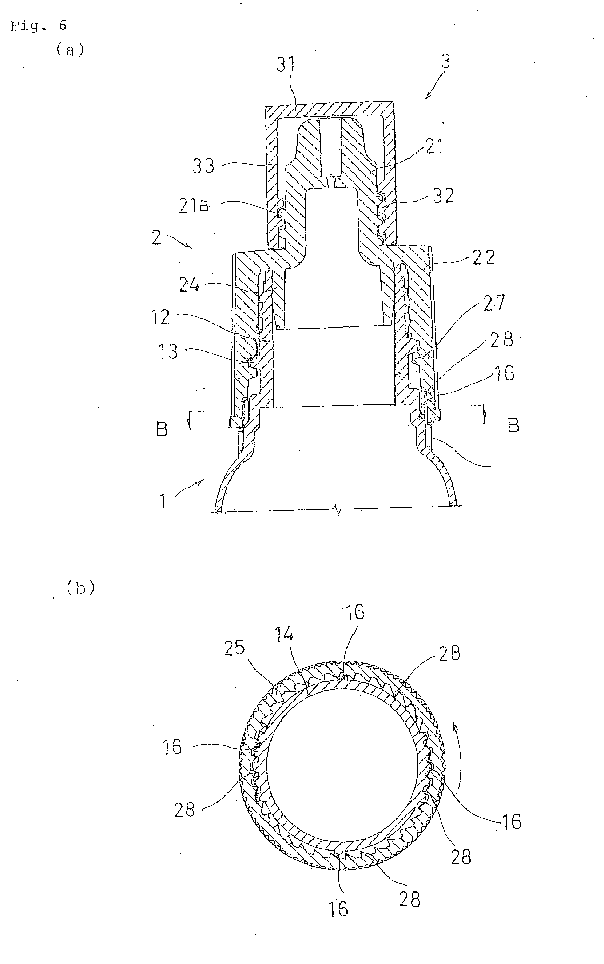

As shown in Figs. 1, 3 and 4, the head section 21 is provided with a male screw 21a on its outer circumferential surface, and is provided with a medicinal solution extracting orifice 21b communicated with an inside of the body section 22 in its apical portion. Further, as shown in Figs. 5 and 6, the cap 3 includes an apical wall 31 and a tube section 33 which is installed vertically downward from the peripheral edge of the apical wall 31 and provided with a male screw 32 screwed together with the male screw 21a of the head section 21 on its inner circumferential surface.

As described above, in the container according to the present invention, the male screw 13 of the container main body 1 and the female screw 27 of the nozzle cap 2, and the male screw 21a of the head section 21 of the nozzle cap 2 and the female screw 32 of the cap 3 are respectively screwed together, as a result, the container main body 1, the nozzle cap 2, and the cap 3 are screwed to each other (refer to Fig. 5).

As shown in Fig. 5, the container according to the present invention is configured so that, in a closed state in which the female screw 27 of the body section 22 of the nozzle cap 2 is screwed together with the male screw 13 of the container main body 1 to be turned up to a position at which the container main body 1 is closed, although the upper stage internal ratchet teeth 28 and the upper stage external ratchet teeth 16 do not engage with each other, the lower stage internal ratchet teeth 29 and the lower stage external ratchet teeth 17 engage with each other to prevent turning in the opening direction (in the direction of an arrow of Fig. 5B). Accordingly, the container main body 1 and the nozzle cap 2 are not loosened before use in any case.

Moreover, as shown in Fig. 6, the container according to the present invention is configured so that, in a closed state in which the sealing ring part 23 is removed from the container main body 1 by breaking the joining sections 41 of the nozzle cap 2, and the female screw 27 of the body section 22 of the nozzle cap 2 without the sealing ring part 23 is screwed together with the male screw 13 of the container main body 1 to be turned up to the position at which the container main body 1 is closed, the upper stage internal ratchet teeth 28 and the upper stage external ratchet teeth 16 engage with each other to prevent turning in the opening direction (in the direction of an arrow of Fig. 6B). Accordingly, provided that the cap 3 is turned in the opening direction (in a counterclockwise direction viewed from above) in this state, the closed state between the nozzle cap 2 and the container main body 1 is kept, which makes it possible to remove only the cap 3.

In use of the container, first, the nozzle cap 2 is operated to turn in the opening direction to remove the nozzle cap 2 from the container main body 1 (first step), next, a second ingredient (not shown) is added into the container main body 1 and the nozzle cap 2 is again operated to turn in the closing direction to close the container main body 1 (second step), and next, the cap 3 is operated to turn in the opening direction to remove the cap 3 from the nozzle cap 2, to discharge a medicinal solution containing the first ingredient and the second ingredient from the medicinal solution extracting orifice 21b (third step).

In the first step, when the nozzle cap 2 is operated to turn in the opening direction (in the direction of an arrow in Fig. 5B), the lower stage internal ratchet teeth 29 and the lower stage external ratchet teeth 17 engage with each other to apply force to prevent turning in the opening direction. Meanwhile, when the nozzle cap 2 is operated to turn in the opening direction against the force, the joining sections 41 are easily broken to allow the nozzle cap 2 to be removed from the container main body 1.

In the second step, after the sealing ring part 23 provided separately from the nozzle cap 2 is removed from the container main body 1 by breaking the joining sections 41 in the first step, the nozzle cap 2 without the sealing ringpart 23 is operated to turn in the closing direction to close the container main body 1.

In the third step, when the cap 3 is turned in the opening direction (in a counterclockwise direction viewed from above), the upper stage internal ratchet teeth 28 and the upper stage external ratchet teeth 16 engage with each other to apply force to prevent turning in the opening direction (in the direction of an arrow of Fig. 6B). Therefore, even when the cap 3 is operated to turn in the opening direction, the closed state between the nozzle cap 2 and the container main body 1 is kept, which makes it possible to remove only the cap 3.

At the time of manufacturing the container according to the present invention, included is a process that the body section 11 of the container main body 1 is filled with a first ingredient, and thereafter, the female screw 27 of the body section 22 of the nozzle cap 2 is screwed together with the male screw 13 of the container main body 1 to be operated to turn in the closing direction, to allow the nozzle cap 2 to be screwed onto the container main body 1. In the above-described process, the turning operation is terminated when the sealing ring part 23 is turned up to a predetermined position shown in Fig. 5A and the lower stage internal ratchet teeth 29 and the lower stage external ratchet teeth 17 engage with each other. However, when the turning operation is further continued, the body section 22 may be excessively turned to break the joining sections 41. Then, in the present invention, the front end of the downward protruding piece 42 comes into contact with the rear end of the upward protruding piece 43 to regulate excessive turning of the body section 22, to perform an appropriate turning operation.

The embodiment of the two-drug pharmaceutical container according to the present invention has been described above. However, the present invention is not limited to only the above-described embodiment. As long as the two-drug pharmaceutical container is configured so that only the lower stage internal and external ratchet teeth 17 and 29 engage with each other to prevent turning in the opening direction in a closed state before use, and is configured so that the upper stage internal and external ratchet teeth 16 and 28 engage with each other to prevent turning in the opening direction in a state in which the container main body is closed with the nozzle cap 2 without the sealing ring part 23, the shapes, the numbers of teeth, the pitches, the positional relationships of height or the like, and the like of the upper and lower stage and internal and external ratchet teeth can be appropriately changed.

### [Industrial Applicability]

The present invention may have broad applicability as a two-drug pharmaceutical container in which its container main body and nozzle cap are not loosened before use, and when the cap is turned in the opening direction at the time of administering eye drops or the like, the nozzle cap and the container main body are not loosened to keep the closed state, which makes it possible to remove only the cap.

### [Description of Reference Numerals]

- 1: Container main body
- 2: Nozzle cap
- 3: Cap
- 12: Nozzle section
- 13: Male screw
- 14: Small diameter annular section
- 15: Large diameter annular section
- 16: Upper stage external ratchet teeth
- 17: Lower stage external ratchet teeth
- 21: Head section
- 21b: Medicinal solution extracting orifice
- 22: Body section
- 23: Sealing ring part
- 25: External tube
- 27: Female screw
- 28: Upper stage internal ratchet teeth
- 29: Lower stage internal ratchet teeth
- 41: Joining sections
- 42: Downward protruding piece
- 43: Upward protruding piece

## Claims

1. A two-drug pharmaceutical container comprising:
a container main body which is filled with a first ingredient, and provided with a male screw on an outer circumferential surface of a nozzle section;
a nozzle cap which has a body section provided with a female screw screwed together with the male screw on an inner circumferential surface of its tube body, a head section including a medicinal solution extracting orifice communicated with an inside of the body section, and a sealing ring part provided via a plurality of breakable joining sections which are provided along a circumferential direction on a lower edge of the body section; and
a cap screwed on so as to cover the head section of the nozzle cap, wherein
a small diameter annular section with an outer diameter larger than an outer diameter of the male screw of the nozzle section and a large diameter annular section, which is on the lower side of the small diameter annular section, with an outer diameter larger than the outer diameter of the small diameter annular section are provided at a lower portion of the nozzle section of the container main body, and upper stage external ratchet teeth and lower stage external ratchet teeth are respectively provided on an outer circumferential surface of the small diameter annular section and an outer circumferential surface of the large diameter annular section, and on the other hand, upper stage internal ratchet teeth and lower stage internal ratchet teeth are respectively provided on an inner circumferential surface of a lower portion of the body section and an inner circumferential surface of the sealing ring part in the components of the nozzle cap,
and in a closed state in which the female screw of the body section of the nozzle cap is screwed together with the male screw of the container main body to be turned up to a position at which the container main body is closed, although the upper stage internal ratchet teeth and the upper stage external ratchet teeth do not engage with each other, the lower stage internal ratchet teeth and the lower stage external ratchet teeth engage with each other to prevent turning in an opening direction, and moreover,
in a closed state in which the joining sections of the nozzle cap are broken to remove the sealing ring part from the container main body, and the female screw of the body section of the nozzle cap without the sealing ring part is screwed together with the male screw of the container main body to be turned up to the position at which the container main body is closed, the upper stage internal ratchet teeth and the upper stage external ratchet teeth engage with each other to prevent turning in the opening direction.

2. The two-drug pharmaceutical container according to claim 1, wherein a downward protruding piece is provided on the lower edge of the body section and an upward protruding piece is provided so as to come close to the closing side in the circumferential direction from the downward protruding piece on an upper edge of the sealing ring part in the components of the nozzle cap, and the front part lower end of the downward protruding piece is extended downward from the rear part upper end of the upward protruding piece.

3. The two-drug pharmaceutical container according to claim 1 or claim 2, the container is used for any purpose of eye drops, ear drops, and nose drops.

4. A method for using the two-drug pharmaceutical container comprising the successive steps of:
in use of the two-drug pharmaceutical container according to any one of claims 1 to 3,
operating the nozzle cap to turn in the opening direction to break the joining sections, and removing the nozzle cap without the sealing ring part and the sealing ring part from the container main body;
adding a second ingredient into the container main body and operating the nozzle cap to turn in the closing direction to close the container main body; and
operating the cap to turn in the opening direction to remove the cap from the nozzle cap, and discharging a medicinal solution containing the first ingredient and the second ingredient from the medicinal solution extracting orifice.
